# EUROPÄISCHE PATENTSCHRIFT

(11) **EP 2 855 402 B1**
(45) Veröffentlichungstag und Bekanntmachung des Hinweises auf die Patenterteilung: **26.09.2018**
(21) Anmeldenummer: 13725640.0
(22) Anmeldetag: 24.05.2013
(51) Int. Cl.: C07B 63/04, C07C 51/50

(54) **VERFAHREN ZUR SOLUBILISIERUNG UND TRENNUNG VON EINER ODER MEHREREN CARBONSÄUREN UND VERWENDUNG EINER DEMOBILISIERTEN SOLUBILISIERENDEN VERBINDUNG**
METHOD FOR SOLUBILISING AND SEPARATING ONE OR A PLURALITY OF CARBOXYLIC ACIDS AND USE OF A DEMOBILISED SOLUBILISING COMPOUND
PROCÉDÉ DE SOLUBILISATION ET DE SÉPARATION D'UN OU PLUSIEURS ACIDES CARBOXYLIQUES ET UTILISATION D'UN COMPOSÉ SOLUBILISANT DÉMOBILISÉ

(30) Priorität: 30.05.2012 DE 102012104668
(43) Veröffentlichungstag der Anmeldung: 08.04.2015
(73) Patentinhaber: GEA Mechanical Equipment GmbH, 59302 Oelde (DE)
(72) Erfinder: HRUSCHKA, Steffen, 59302 Oelde (DE); BOSZULAK, Wladislawa, 59302 Oelde (DE)
(74) Vertreter: Specht, Peter
(86) Internationale Anmeldenummer: PCT/EP2013/060777
(87) Internationale Veröffentlichungsnummer: WO 2013/178551

(56) Entgegenhaltungen:
- WO-A2-2011/160857

## Beschreibung

Die Erfindung betrifft ein Verfahren nach den Merkmalen i) und ii) des Anspruchs 1 und eine Verwendung.

Ein solches Verfahren ist aus der WO 2011/160857 A2 bekannt. Besonders bevorzugt wird bei dem in dieser Schrift offenbarten Verfahren zur Solubilisierung bzw. Katalyse einer Arginin-Wasserlösung verwendet.

Hinsichtlich der Einzelheiten, insbesondere hinsichtlich der Definition von Arginin und deren Derivate, wird vollumfänglich auf die Offenbarung der WO 2011/160857 A2, insbesondere S.24-26 der WO 2011/160857 A2, verwiesen.

Ein noch nicht gelöstes Problem des Verfahrens besteht in dem relativ hohen Kostenaufwand zum Bereitstellen der solubilisierenden Verbindung (Katalysator), insbesondere des Arginins zum Herstellen der Arginin-Wasserlösung.

Die Erfindung hat die Aufgabe, dieses Problem zu beheben.

Die Erfindung löst diese Aufgabe durch den Gegenstand des Anspruchs 1.

Erfindungsgemäß wird dabei das Arginin (Arg) ganz oder teilweise wiedergewonnen.

Analog zu den aus der WO 2011/160857 A2 bekannten Verfahren erfolgt in einem ersten Schritt das Bereitstellen einer Lösung oder Emulsion oder Suspension aus Öl mit Carbonsäuren, welche im Detail in der WO 2011/160857 A2 genannt sind. Im Anschluss daran erfolgt nach der Erfindung ein Zugeben einer Menge, vorzugsweise - aber nicht zwingend - einer mindestens äquimolaren Menge mindestens einer solubilisierenden Verbindung, insbesondere von Arginin oder eines Argininderivates zu der Lösung, Suspension oder Emulsion.

Dabei ist unter Arginin sowohl L-Arginin, D- Arginin oder auch ein racemisches Gemisch beider Verbindungen zu verstehen, sowie deren Derivate. Zur weiteren Definition von Arginin oder deren Derivate wird auf S.24-26 der WO2011/160857 A2 verwiesen. Im Unterschied zur WO2011/160857 A2 erfolgt allerdings die Zugabe des Arginins in Form einer demobilisierten Verbindung.

Dabei stellt sich ein pH-Wert oberhalb von pH=9, vorzugsweise bei pH=11, ein und es bildet sich eine Miszelle aus Wassermikrotropfen um das demobilisierte Arginin oder deren Derivat aus. Diese Wassermiszelle kann aus der Ölphase aussedimentieren und als Wasserphase zentrifugal abgetrennt werden.

Sodann erfolgt die Solubilisierung der Carbonsäure oder der Carbonsäurederivate und das Abtrennen der solubilisierten Carbonsäuren oder -derivate als entsprechende Phase aus der Lösung, Emulsion oder Suspension. Unter einer solubilisierenden Verbindung ist im Rahmen dieser Anmeldung als katalytische Verbindung zu bezeichnen bzw. als Solubilisierung ein Katalyseverfahren. Schließlich erfolgt erfindungsgemäß ein Wiedergewinnen zumindest einer Teilmenge der solubilisierenden Verbindung oder einer Teilmenge eines Derivates der solubilisierenden Verbindung, des Arginins oder des Argininderivates, aus den solubilisierten und als Phase abgetrennten Carbonsäuren oder Carbonsäurederivaten.

Diese Mehrfachverwendung bzw. Rückgewinnung von Arginin ermöglicht einen wirtschaftlichen Umgang mit dem Arginin und senkt den Kostenaufwand des Verfahrens insgesamt.

Es ist zudem erfindungsgemäß, wenn eine Änderung des pH-Wertes bei der Wiedergewinnung auf einen Wert von weniger als pH=7,5 erfolgt. Durch das Absenken des pH-Wertes wird die Bindung zwischen der Carbonsäure und dem Arginin soweit geschwächt, dass eine zusätzliche Verbesserung der Abtrennung und Rückgewinnung des Arginins, vorzugsweise durch Filtration oder zentrifugale Trennung möglich ist. Ein Derivat kann in diesem Zusammenhang beispielsweise eine protonierte Spezies des Arginin sein.

Vorteilhafte Ausgestaltungen der Erfindung sind den Unteransprüchen zu entnehmen. Die Erfindung schafft zudem die Verwendung des Anspruchs 12.

Es ist von Vorteil, wenn als solubilisierende Verbindung eine demobilisierte solubilisierende Verbindung verwendet wird. Dadurch wird die Rückgewinnung wesentlich vereinfacht, da durch die Lokalisierung der solubilisierenden Verbindung eine schwerere Komponente geschaffen wird, welche sich besser durch zentrifugale Trennung aus der Carbonsäurephase- bzw. lösung entfernen lässt.

Dabei weist die demobilisierte solubilisierende Verbindung eine Reaktivkomponente auf, welche die Solubilisierung einer Carbonsäure, vorzugsweise einer Fettsäure ermöglicht.

Diese Reaktivkomponente ist erfindungsgemäß Arginin oder ein Argininderivat. Entsprechende Reaktivkomponenten, welche für eine solubilisierende Verbindung in Frage kommen sind hinreichend im Kapitel "solvation and adehsion behaviour of fatty acids in aqueous media" der WO 2011/160857 A2 beschrieben.

Im Unterschied dazu weist eine demobilisierte solubilisierende Verbindung vorzugsweise zusätzlich ein Trägermaterial auf, auf welchem die solubilisierende Verbindung aufgebracht ist.

Dieses Trägermaterial ist vorzugsweise als Granulat oder Kugelförmig ausgestaltet, um der Reaktivkomponente eine möglichst große Oberfläche zur Anlagerung zu bieten.

Das erfindungsgemäße Verfahren wird im Anschluss im Detail näher erläutert:
Ausgehend von der WO2011/160857 A2 wird vorzugsweise eine Wasser/Arginin-Lösung in ein Öl mit freien Fettsäuren und Phosphatiden zugegeben. Das Arginin oder Argininderivat hat einen pH-Wert von zumindest 9, vorzugsweise 11. Nach dem Schleudern des Gemisches bleibt eine Arginin-Fettsäure-Phosphatid-Wasserphase als schwere Phase zurück, aus welcher zusätzlich zur freien Fettsäure auch das Arginin aufgearbeitet werden soll.

Dabei könnte man zur Rückgewinnung des Arginins gemäß der WO 2011/160857 A2 Wasser verdampfen, was kostenintensiv ist. Alternativ könnte man das Arginin abfiltrieren unter Verwendung eines Siebes aus einem immobilisierten Katalysators. Für das Sieben bzw. Filtrieren könnte man das Arginin zu größeren Einheiten zusammenfassen.

Eine bevorzugte Wiedergewinnung des Arginins kann nach ersten bevorzugten Reaktionsbedingungen durch eine pH-Absenkung durch Zugabe einer Säure, vorzugsweise Phosphorsäure erfolgen. Dabei wird der pH-Wert vorzugsweise auf einen pH-Wert von weniger als 2, insbesondere auf pH=1 oder weniger gesenkt.

Nach der pH-Wert Absenkung ergibt sich eine kontinuierliche Wasserphase mit suspendierten, feinstdispergierten Arginin einerseits und einem Gelee aus freier Fettsäure und Phosphatiden andererseits.

Anschließend kann eine Trennung, beispielsweise durch Filtration erfolgen, wobei eine wässrige Argininphase zurückbleibt. Im Anschluss daran wird der pH-Wert dann wiederum durch eine Neutralisation vorzugsweise mit Lauge, insbesondere mit NaOH in den basischen Bereich von über pH=9, vorzugsweise auf pH 11, eingestellt. Das entstandene Salz ist abzutrennen.

Nach zweiten bevorzugten Reaktionsbedingungen für die Wiedergewinnung des Arginins erfolgt eine Zugabe von Alkohol auf die Arginin-Fettsäure-Phosphatid-Wasserphase und Einstellung eines pH-Wertes von weniger als 7,5, insbesondere von pH=5-6.

Anschließend kann die freie Fettsäure-Phosphatidphase als leichte Phase abzentrifugiert werden. Zurück bleibt eine wässrig-alkoholische Argininlösung mit einem pH-Wert von vorzugsweise 5-6, welcher der Alkohol zu entziehen ist. Im Anschluss wird der pH-Wert wiederum auf einen pH-Wert von über pH=9, vorzugsweise pH=11 eingestellt.

Durch die vorgenannten Varianten der Abtrennung des Arginins steht dieses als solubilisierende Verbindung für weitere Solubilisierungen von Carbonsäuren bereit.

Beide und andere denkbare Spaltungen, führen allerdings immer zu einer wässrigen Arginin-Feinstdispersion mit relativ viel Wasser. Die vorgenannten Reaktionsbedingungen der ersten und zweiten Ausführungsvariante stellen bevorzugte Reaktionsbedingungen des nachfolgenden erfindungsgemäßen Verfahrens dar. Nachteilig ist allerdings der Bedarf an großen Mengen Flüssigkeit, Säuren, Laugen und die resultierende dünne Arginin-Lösung.

Es ist daher ein Vorteil der vorliegenden Erfindung, dass das Arginin relativ trocken aus der Wasserphase, besonders bevorzugt durch zentrifugale Trennung zurückzugewinnen ist.

Besonders bevorzugt geschieht dies durch die Spaltung in
a) eine freie Fettsäure-Phosphatid-Phase
b) eine Wasserphase
c) eine Arginin-Trägermittel-Phase (Arginingranulat) .

Hierzu wird nach einer vorteilhaften Weiterbildung des erfindungsgemäßen Verfahrens, ein Arginin-Granulat bereitgestellt, das dann als Arginin-Trägermittel-Phase c) relativ trocken abgetrennt werden kann. Zur Wiederverwertung ist es daher besonders von Vorteil, Arginin oder ein Argininderivat in irgendeiner Form auf einen festen Träger zu bringen, wodurch das Arginin demobilisiert wird.

Hierzu wird Arginin vorzugsweise vor der Zugabe zur Carbonsäure, insbesondere der freien Fettsäure, auf PVPP (Polyvinylpyrrolidon), insbesondere Divergan, gemäß der Produktspezifikation der BASF zum Zeitpunkt der Anmeldung, aufgebracht. Dies erfolgt vorzugsweise durch Aufsprühen des wässrigen Arginins auf das PVPP-Trägermaterial.

Dabei haftet die Carboxylgruppe des Arginins auf dem mikroskopisch zerklüfteten PVPP, vorzugsweise dem Divergan F, an und die Guanidingruppe zur Reaktion mit der Fettsäure verbleibt als aktive Gruppe an der Oberfläche des Trägermaterials. Der pH-Wert der Aminosäure ist dabei vorzugsweise zwischen 9-10.

Im Anschluss erfolgt die Zugabe des auf dem Trägermaterial aufgebrachten Arginins in das Öl bzw. zur Carbonsäurelösung, -suspension oder -emulsion. Diese Zugabe erfolgt durch Zugabe von Wasser. Dabei wird die freie Fettsäure, wie bereits zuvor beschrieben, solubilisiert und als freie Fettsäure-Phosphatid-Phase a), vorzugsweise zentrifugal, aus der Lösung entfernt.

Dabei ist die Gegenwart einer Mindestmenge von Wasser für die Solubilisierung essentiell, da andernfalls keine Abtrennung der Fettsäuren aus einem Öl mittels der solubilisierenden Verbindung möglich wäre. Das Wasser kann bereits z.T. im Öl enthalten sein, beispielsweise als kolloidale Lösung. Sollte diese Menge nicht ausreichen, so kann noch zusätzlich Wasser nachdosiert werden.

Im Anschluss oder gleichzeitig mit der Abtrennung der freien Fettsäure-Phosphatid-Phase a) erfolgt die Abtrennung des wässrigen Granulates bzw. des mit Arginin-versehenen Trägermaterials. Dabei ist vorzugsweise der pH-Wert auf einen Wert von weniger als 7,5, vorzugsweise 5-6 zu senken, so dass die freien Fettsäuren (free fatty acids = FFA) ihre Bindung zum Arginin verlieren und das Arginin-PVPP Granulat abgetrennt werden kann. Dies kann ggf. mit Unterstützung von Alkohol erfolgen, wie dies bereits im oben-beschriebenen zweiten Ausführungsbeispiel erläutert wurde.

Nach einer Waschung ggf. Trocknung (auch Teilweise) kann das auf dem Trägermaterial demobilisierte Arginin wiederverwendet werden.

Besonders bevorzugt ist dabei die Gegenwart von Wasser. Die alleinige Zugabe des Granulat in ein Öl reicht nicht, es muss in Wasser aufgelöst oder zumindest suspendiert sein. Dadurch wird eine Abtrennung des Arginin-Granulates mit den freien Fettsäuren und den Phosphatidgruppen aus einer Ölphase ermöglicht.

Bei Einstellung der Wasserphase gemäß den oben-genannten bevorzugten ersten Reaktionsbedingungen bilden die Komplexe aus PVPP-Arginin, die freien Fettsäuren und die Phospholipide zusammen mit Wasser eine Miszelle in der Ölphase bzw. Mikrowassertröpfchen aus, welche sich aus der Ölphase heraus absetzen und eine abschleuderbare Wasserphase bilden.

Bei Einstellung der Wasserphase gemäß den oben-genannten bevorzugten zweiten Reaktionsbedingungen, beispielsweise für eine 15%ige Ethanollösung bei pH=5,0 hat zudem eine Feintrennung in eine Phospholipid/Fettsäurephase und eine Phase aus demobilisierten Arginin ergeben, wobei die Arginin-PVPP Phase als schwerere Phase aus dem Phospholipid/Fettsäuregemisch aussedimentiert.

Zusätzlich mit der Wasserphase ergibt sich nach der Abtrennung aus der Ölphase somit ein Dreiphasensystem aus einer klaren ethanolisch-wässrigen Lösung, einem Gemisch aus Phospholipiden und freien Fettsäuren und der Arginin-PVPP-Phase, die als Kuchen mit geringem Feuchtigkeitsgehalt abgetrennt werden kann.

Alternativ zur Demobilisierung des Arginins auf einem Trägermaterial kann auch die Demobilisierung des Arginins in einer zweiten Ausführungsvariante des erfindungsgemäßen Verfahrens durch Umwandlung in ein Argininderivat - vorzugsweise eines Peptides - und anschließendes Aufbringen auf ein Trägermaterial erfolgen.

Hierbei erfolgt zunächst die Herstellung eines Peptides aus Arginin unter Zugabe einer vorzugsweisen schwefelhaltigen Aminosäure wie Cystein oder Methionin. Dieses Di-Amino-Peptid, bei dem sich die Guanidingruppe des entstandenen Peptids mit der freien Fettsäure verbindet, kann sich an einem Trägermaterial - vorzugsweise an Stahlkügelchen anlagern. Das Eisen im Stahl verbindet sich mit den funktionellen Gruppen der Aminosäure, vorzugsweise der schwefelhaltigen Aminosäure. Es bildet sich somit ein Stahl-Aminosäure-Arginin-Komplex aus zu welchem ebenfalls Wasser zugegeben wird, soweit dieses nicht bereits in der Ölphase suspendiert vorliegt. Dabei bildet sich abermals eine Miszelle aus dem besagten Komplex, der freien Fettsäure, Phospholipid und Wasser aus, welche sich in der Ölphase absetzt und eine wäßrige Phase bildet.

Wie bereits beschrieben, lässt dich die Komplexbildung gezielter kontrollieren, wenn zunächst der Arginin-Aminosäurekomplex gebildet wird und anschließend dieser auf der Oberfläche der Stahlkügelchen aufgebracht wird. Dabei bildet die Aminosäure die Verbindung zwischen der jeweiligen Stahlkugel und dem Argininmolekül aus, ohne die Funktionalität des Arginins zu beeinflussen.

Auch auf diese Ausführungsvariante können die oben-genannten ersten oder zweiten Reaktionsbedingungen bevorzugt angewandt werden, wobei bei der zweiten Reaktionsbedingung, beispielsweise bei einer 15%igen Ethanollösung, bei pH=5 ein Sedimentieren des Komplexes der Stahlkügelchen aus dem Gemisch aus freien Fettsäuren und Phospholipiden erfolgt. Nach der 3-Phasentrennung erhält man eine saubere ethanolisch-wässrige Lösung, eine Phospholipid-Fettsäurelösung und ein Kuchen des Komplexes mit geringer Restfeuchtigkeit.

Die Zugabe und Rückgewinnung dieser Kombination des Arginindetivates und des Trägermaterials im Solubilisierungsverfahren erfolgt analog zur oben-genannten Ausführungsvariante des PVPP-Granulates.

Untersuchungen bezüglich der zweiten Ausführungsvariante der Domobilisierung haben gezeigt, dass das Duplex- und ferritische Stähle eine deutlich bessere Proteinanhaftung aufweisen als Austenite. Außerdem hängt die Anhaftung von der unterschiedlichen Zusammensetzung der Proteine ab. So werden einzelne Aminosäuren besser kleben als andere. Wichtig hierbei ist die HS-Gruppe bzw. die Schwefelgruppe der Proteine.

Diese verbindet sich vorzugsweise mit dem Eisen des Stahles. Es wurde ferner gefunden, dass stickstoffhaltige Stähle eine bessere Anhaftung für die oben-genannten Argininderivate bieten als Stähle mit einem vernachlässigbar kleinem Anteil an Stickstoff. Daher ist die Verwendung von stickstoffhaltigen Stählen als Trägermaterial bevorzugt.

Ferner spielt die Oberflächenstruktur der Stähle eine Rolle. Nach dem Warmwalzen der Stähle werden diese gebeizt, so dass an der Oberfläche der Stähle Löcher bzw Poren kleiner von 1 Mikrometer (Mikroporosität) entstehen, in die sich Peptide oder Aminosäuren anlagern können. Daher ist die Verwendung von Stählen mit mikroporösen Strukturen, d.h. von Strukturen mit Löcher mit einem durchschnittlichen Durchmesser von kleiner als 1 Mikrometer, als Trägermaterial bevorzugt. Insbesondere die Verwendung von warmgewalzten Stählen.

Es ist darüber hinaus auch möglich, zunächst geeignete Aminosäuren auf einer Metall - insbesondere Stahloberfläche zu demobilisieren und im Anschluss unter Zugabe des Arginins ein entsprechendes Argininderivat zu schaffen.

Die Kombination aus geeignete Aminosäuren oder Peptiden, beispielsweise Cystein oder Thiamin, mit einer guter Klebefähigkeit auf den o.g., vorzugsweise nichtaustenidischen, Stählen mit funktionellen Aminosäuren, insbesondere Arginin, oder Peptiden, insbesondere Enzymen, ermöglicht eine unkomplizierte Rückgewinnung und Mehrfachverwendung der solubilisierenden Verbindung, respektive des Arginins oder des Argininderivates.

So kann man anschließend diese Kombiprodukte auf den Stahlen oder Stahlpulvern mit der geeigneten mikroporosen Oberflächen kleben lassen. Man erhält so stahlbeschwerte funktionelle Produkte wie Aminosäuren, die auf Stahloberflächen geklebt sind und so spezifisch schwerer als Öl oder Wasser werden und sich zentrifugal gut aus dem o.g. Phasengemisch entfernen lassen.

## Patentansprüche

1. Verfahren zur Solubilisierung und Trennung einer oder mehrere Carbonsäuren oder Carbonsäurederivaten aus einer wässrigen oder organischen Lösung, Emulsion oder Suspension, umfassend die folgenden Schritte:
i) Bereitstellen der Lösung oder Emulsion oder Suspension mit der Carbonsäure oder den Carbonsäurederivaten;
ii) Zugeben einer Menge einer solubilisierenden Verbindung, in Form von Arginin oder einem Argininderivat, zu der Lösung, Suspension oder Emulsion in Gegenwart einer Mindestmenge an Wasser, wobei als solubilisierende Verbindung eine demobilisierte solubilisierende Verbindung verwendet wird;
iii) Abtrennen der solubilisierten Carbonsäuren oder Carbonsäusederivate als eine Carbonsäure- oder Carbonsäurederivatephase aus der Lösung oder Emulsion oder Suspension, und
iv) Wiedergewinnen zumindest einer Teilmenge der solubilisierenden Verbindung oder einer Teilmenge eines Derivates der solubilisierenden Verbindung, in Form des Arginins oder des Argininderivates, aus der solubilisierten und abgetrennten Carbonsäurephase oder Carbonsäurederivatephase;
wobei die Wiedergewinnung in Schritt iv) durch Änderung des pH-Wertes der solubilisierten Carbonsäuren oder Carbonsäurederivatephase auf einen Wert von weniger als pH=7,5 erfolgt.

2. Verfahren nach Anspruch 1, **dadurch gekennzeichnet, dass** die demobilisierte solubilisierende Verbindung eine Reaktivkomponente aufweist, welche die Solubilisierung einer Carbonsäure, vorzugsweise einer Fettsäure ermöglicht und ein Trägermaterial aufweist, auf welches die solubilisierende Verbindung aufgebracht ist.

3. Verfahren nach Anspruch 2, **dadurch gekennzeichnet, dass** das Trägermaterial ein PVPP-Granulat oder Zheolit oder Kieselgur oder einer sonstigen Diatomerde oder Bentonit oder dgl. ist.

4. Verfahren nach einem der vorhergehenden Ansprüche, **dadurch gekennzeichnet, dass** die Reaktivkomponente der demobilisierte solubilisierende Verbindung ein Peptid ist, hergestellt aus Arginin oder einem Argininderivat und einer Aminosäure, vorzugsweise einer schwefelhaltigen Aminosäure

5. Verfahren nach Anspruch 2, **dadurch gekennzeichnet, dass** als Trägermaterial ein Metall, vorzugsweise ein Stahl, besonders bevorzugt ein nichtaustenidischer Stahl verwendet wird.

6. Verfahren nach einem der vorhergehenden Ansprüche, **dadurch gekennzeichnet, dass** als Reaktivkomponente ein Arginin oder ein Argininderivat verwendet wird.

7. Verfahren nach einem der vorhergehenden Ansprüche, **dadurch gekennzeichnet, dass** das Abtrennen der solubilisierten Carbonsäure- oder Carbonsäurederivatephase aus der Lösung oder Emulsion oder Suspension in Form einer Arginin-Fettsäure-Phosphatid-Wasserphase erfolgt.

8. Verfahren nach Anspruch 7, **dadurch gekennzeichnet, dass** durch das Absenken des pH-Wertes auf weniger als pH=7,5, vorzugsweise auf pH=5-6, die Bindung zwischen der Carbonsäure oder den Carbonsäurederivaten und der solubilisierenden Verbindung geschwächt oder aufgehoben wird.

9. Verfahren nach Anspruch 8, **dadurch gekennzeichnet, dass** durch das Absenken des pH-Wertes auf bis maximal pH = 2 erfolgt..

10. Verfahren nach Anspruch 8 oder 9, **dadurch gekennzeichnet, dass** ergänzend ein polares Lösungsmittel bei der pH-Wert Absenkung zugegeben wird.

11. Verfahren nach einem der vorhergehenden Ansprüche, **dadurch gekennzeichnet, dass** das Wiedergewinnen, gemäß Schritt iv, durch zentrifugale Trennung der vorzugsweise demobilisierten, solubilisierten Verbindung aus der solubilisierten und abgetrennten Carbonsäurephase oder Carbonsäurederivatephase, insbesondere der Arginin-Fettsäure-Phosphatid-Wasserphase erfolgt.

12. Verwendung einer demobilisierten solubilisierenden Verbindung in einem Verfahren zur Solubilisierung und Trennung von einer oder mehreren Carbonsäuren oder Carbonsäurederivaten aus einer wässrigen oder organischen Lösung, Emulsion oder Suspension, umfassend die folgenden Schritte:
i) Bereitstellen der Lösung oder Emulsion oder Suspension mit den Carbonsäuren oder Carbonsäurederivaten;
ii) Zugeben einer Menge von Arginin oder einem Argininderivat als solubilisierende Verbindung zu der Lösung, Suspension oder Emulsion in Gegenwart einer Mindestmenge an Wasser,
iii) Abtrennen der solubilisierten Carbonsäurephase oder Carbonsäurederivatephase aus der Lösung oder Emulsion oder Suspension,
iv) wobei die demobilisierte solubilisierenden Verbindung nach dem Abtrennen der solubilisierten Carbonsäurephase oder Carbonsäurederivatephase, gemäß Schritt iii) aus der solubilisierten Carbonsäurephase oder Carbonsäurederivatephase zurückgewinnbar ist;
wobei die Wiedergewinnung in Schritt iv) durch Änderung des pH-Wertes der solubilisierten Carbonsäuren oder Carbonsäurederivatephase auf einen Wert von weniger als pH=7,5 erfolgt.

## Claims

1. A method for solubilizing and separating one or more carboxylic acids or carboxylic acid derivatives from an aqueous or organic solution, emulsion or suspension, comprising the following steps:
i) providing the solution or emulsion or suspension with the carboxylic acid or the carboxylic acid derivatives;
ii) adding an amount of a solubilizing compound,
in the form of arginine or an arginine derivative, to the solution, suspension or emulsion in the presence of a minimum amount of water, wherein the solubilizing compound used is an immobilized solubilizing compound;
iii) separating the solubilized carboxylic acids or carboxylic acid derivatives from the solution or emulsion or suspension as a carboxylic acid or carboxylic acid derivative phase, and
iv) recovering at least a portion of the solubilizing compound or a portion of a derivative of the solubilizing compound, in the form of the arginine or the arginine derivative, from the solubilized and separated carboxylic acid phase or carboxylic acid derivative phase;
wherein the recovery in step iv) takes place by changing the pH of the solubilized carboxylic acids or carboxylic acid derivative phase to a value of less than pH = 7.5.

2. The method as claimed in claim 1, **characterized in that** the immobilized solubilizing compound has a reactive component which enables the solubilization of a carboxylic acid, preferably a fatty acid, and has a support material to which the solubilizing compound is applied.

3. The method as claimed in claim 2, **characterized in that** the support material is a granulate of PVPP or zeolite or kieselguhr or another diatomaceous earth or bentonite or the like.

4. The method as claimed in any of the preceding claims, **characterized in that** the reactive component of the immobilized solubilizing compound is a peptide prepared from arginine or an arginine derivative and an amino acid, preferably a sulphur-containing amino acid.

5. The method as claimed in claim 2, **characterized in that** the support material used is a metal, preferably a steel, particularly preferably a nonaustenitic steel.

6. The method as claimed in any of the preceding claims, **characterized in that** the reactive component used is an arginine or an arginine derivative.

7. The method as claimed in any of the preceding claims, **characterized in that** the solubilized carboxylic acid or carboxylic acid derivative phase is separated from the solution or emulsion or suspension in the form of an arginine-fatty acid-phosphatide-water phase.

8. The method as claimed in claim 7, **characterized in that** the bond between the carboxylic acid or the carboxylic acid derivatives and the solubilizing compound is weakened or broken by reducing the pH to less than = 7.5, preferably to a pH = 5-6.

9. The method as claimed in claim 8, **characterized in that** the pH is reduced up to a maximum pH = 2.

10. The method as claimed in claim 8 or 9, **characterized in that**, in addition, a polar solvent is added during the lowering of the pH.

11. The method as claimed in any of the preceding claims, **characterized in that** the recovery, according to step iv, is carried out by centrifugal separation of the preferably immobilized, solubilized compound from the solubilized and separated carboxylic acid phase or carboxylic acid derivative phase, in particular the arginine-fatty acid-phosphatide-water phase.

12. The use of an immobilized solubilizing compound in a method for solubilizing and separating one or more carboxylic acids or carboxylic acid derivatives from an aqueous or organic solution, emulsion or suspension, comprising the following steps:
i) providing the solution or emulsion or suspension with the carboxylic acids or the carboxylic acid derivatives;
ii) adding an amount of arginine or an arginine derivative as a solubilizing compound to the solution, suspension or emulsion in the presence of a minimum amount of water,
iii) separating the solubilized carboxylic acid phase or carboxylic acid derivative phase from the solution or emulsion or suspension,
iv) wherein the immobilized solubilizing compound, after separating the solubilized carboxylic acid phase or carboxylic acid derivative phase according to step iii), may be recovered from the solubilized carboxylic acid phase or carboxylic acid derivative phase;
wherein the recovery in step iv) takes place by changing the pH of the solubilized carboxylic acids or carboxylic acid derivative phase to a pH of less than = 7.5.

## Revendications

1. Procédé pour solubiliser et séparer un ou plusieurs acides carboxyliques ou dérivés d'acide carboxylique à partir d'une solution, émulsion ou suspension aqueuse ou organique, comprenant les étapes suivantes :
i) préparation de la solution, émulsion ou suspension contenant l'acide carboxylique ou les dérivés d'acide carboxylique ;
ii) ajout d'une quantité d'un composé solubilisant, sous forme d'arginine ou d'un dérivé d'arginine, à la solution, suspension ou émulsion en présence d'une quantité minimale d'eau, le composé solubilisant utilisé étant un composé solubilisant démobilisé ;
iii) séparation des acides carboxyliques ou dérivés d'acide carboxylique solubilisés sous la forme d'une phase d'acides carboxyliques ou de dérivés d'acide carboxylique à partir de la solution, émulsion ou suspension, et
iv) récupération d'au moins une partie du composé solubilisant ou d'une partie d'un dérivé du composé solubilisant, sous la forme de l'arginine ou du dérivé d'arginine, à partir de la phase d'acides carboxyliques ou de dérivés d'acide carboxylique solubilisée et séparée ;
dans lequel la récupération dans l'étape iv) est effectuée en modifiant le pH de la phase d'acides carboxyliques ou de dérivés d'acide carboxylique solubilisée à une valeur inférieure à pH = 7,5.

2. Procédé selon la revendication 1, **caractérisé en ce que** le composé solubilisant démobilisé comprend un composant réactif qui permet la solubilisation d'un acide carboxylique, de préférence d'un acide gras, et présente un matériau de support sur lequel le composé solubilisant est appliqué.

3. Procédé selon la revendication 2, **caractérisé en ce que** le matériau de support est formé de granulés de PVPP ou de zéolithe ou de kieselguhr ou d'une autre diatomite ou de bentonite ou similaire.

4. Procédé selon l'une des revendications précédentes, **caractérisé en ce que** le composant réactif du composé solubilisant démobilisé est un peptide fabriqué à partir d'arginine ou d'un dérivé d'arginine et d'un acide aminé, de préférence d'un acide aminé soufré.

5. Procédé selon la revendication 2, **caractérisé en ce que** le matériau de support utilisé est un métal, de préférence un acier, en particulier un acier non austénitique.

6. Procédé selon l'une des revendications précédentes, **caractérisé en ce que** le composant réactif utilisé est une arginine ou un dérivé d'arginine.

7. Procédé selon l'une des revendications précédentes, **caractérisé en ce que** la séparation de la phase d'acides carboxyliques ou de dérivés d'acide carboxylique solubilisée à partir de la solution, émulsion ou suspension s'effectue sous la forme d'une phase aqueuse d'arginine, acides gras et phosphatides.

8. Procédé selon la revendication 7, **caractérisé en ce que** l'abaissement du pH à moins de pH = 7,5, de préférence à pH = 5-6, affaiblit ou abolit la liaison entre l'acide carboxylique ou les dérivés d'acide carboxylique et le composé solubilisant.

9. Procédé selon la revendication 8, **caractérisé en ce que** le pH est abaissé jusqu'à pH = 2 au maximum.

10. Procédé selon la revendication 8 ou 9, **caractérisé en ce qu'**un solvant polaire est ajouté en complément lors de l'abaissement du pH.

11. Procédé selon l'une des revendications précédentes, **caractérisé en ce que** la récupération selon l'étape iv) est effectuée par séparation centrifuge du composé solubilisé et de préférence démobilisé formé par la phase d'acides carboxyliques ou de dérivés d'acide carboxylique solubilisée et séparée, en particulier la phase aqueuse d'arginine, acides gras et phosphatides.

12. Utilisation d'un composé solubilisant démobilisé dans un procédé pour solubiliser et séparer un ou plusieurs acides carboxyliques ou dérivés d'acide carboxylique à partir d'une solution, émulsion ou suspension aqueuse ou organique, comprenant les étapes suivantes :
i) préparation de la solution, émulsion ou suspension contenant l'acide carboxylique ou les dérivés d'acide carboxylique ;
ii) ajout d'une quantité d'arginine ou d'un dérivé d'arginine à la solution, suspension ou émulsion pour servir de composé solubilisant, en présence d'une quantité minimale d'eau ;
iii) séparation des acides carboxyliques ou dérivés d'acide carboxylique solubilisés à partir de la solution, émulsion ou suspension,
iv) le composé solubilisant démobilisé pouvant être récupéré, après la séparation de la phase d'acides carboxyliques ou de dérivés d'acide carboxylique solubilisées selon l'étape iii), à partir de la phase d'acides carboxyliques ou de dérivés d'acide carboxylique solubilisée ;
la récupération dans l'étape iv) étant effectuée par modification du pH de la phase d'acides carboxyliques ou de dérivés d'acide carboxylique solubilisée à une valeur inférieure à pH = 7,5.
